# EUROPEAN PATENT SPECIFICATION

(11) **EP 2 726 903 B1**
(45) Date of publication and mention of the grant of the patent: **08.08.2018**
(21) Application number: 12741385.4
(22) Date of filing: 28.06.2012
(51) Int. Cl.: G01S 15/89, G01S 7/52

(54) **TWO DIMENSIONAL ULTRASONIC DIAGNOSTIC IMAGING SYSTEM WITH TWO BEAMFORMER STAGES**
ZWEIDIMENSIONALES ULTRASCHALLDIAGNOSE- UND BILDGEBUNGSSYSTEM MIT ZWEI STRAHLFORMERSTUFEN
SYSTÈME D'IMAGERIE DE DIAGNOSTIC ULTRASONORE BIDIMENSIONNEL PRÉSENTANT DEUX ÉTAGES DE FORMEUR DE FAISCEAU

(30) Priority: 30.06.2011 US 201161503329 P
(43) Date of publication of application: 07.05.2014
(73) Proprietor: Koninklijke Philips N.V., 5656 AE Eindhoven (NL)
(72) Inventor: POLAND, McKee, Dunn, NL-5656 AE Eindhoven (NL); ROBINSON, Andrew, Lee, NL-5656 AE Eindhoven (NL)
(74) Representative: Steffen, Thomas
(86) International application number: PCT/IB2012/053281
(87) International publication number: WO 2013/001484

(56) References cited:
- WO-A1-2010/055428
- DE-A1-102008 044 423
- DE-C1- 19 741 361
- US-A1- 2002 035 328
- US-A1- 2005 148 873
- US-B1- 6 500 126

## Description

This invention relates to ultrasonic medical diagnostic imaging systems which perform two dimensional (2D) imaging and, in particular, to 2D ultrasonic diagnostic systems with two beamformer stages.

Medical diagnostic imaging systems with multielement solid state probes employ a beamformer to steer and focus beams. Traditionally the beamformer is in the electronics compartment of the mainframe system and is coupled to the transducer array of the probe through the probe cable. Each channel of the beamformer is coupled to one of the elements of the probe's array transducer. On transmit, the beamformer channels provide properly timed transmit signals to the elements which cause the transmit beam to be steered in a desired direction and focused at a desired depth. During reception of the resulting echo signals, the process is reversed. Each channel appropriately delays the echo signals from its transducer element so that, when the echo signals from all of the channels are combined, the receive beam is steered and focused in the desired direction and depth, generally that of the transmit beam. The probe and beamformer can thus scan the image field with a series of adjacent beams to form a 2D image of an image plane of the body.

Beamformers are also used to scan and receive beams over a volumetric region to form a 3D image of the region. To steer the beams in elevation as well as azimuth a two dimensional array transducer is used. The typical two dimensional array for 3D imaging has many times the number of elements of the single row of a 2D imaging probe, generally numbering in the thousands. This presents two problems. One is that a cable with thousands of wires from the system beamformer to the transducer elements would make the cable very thick and impractical. The other is that a significant amount of power is expended in driving the thousands of elements with transmit signals, causing excessive heat in the probe. These two problems have been addressed by the development of probe microbeamformers, as described in US Pat. 5,229,933 (Larson III). As shown on the cover of the patent and described therein, the initial part of the beamforming is done in the probe itself. Since the probe must be easily manipulated by a sonographer, the beamformer in the probe must be small and light and thus is formed of integrated circuitry. The integrated circuitry consumes much less power than the system beamformer, thereby resolving the heating problem. The many elements of the two dimensional array transducer are connected to channels of the microbeamformer, which partially beamforms the many signals down to a lesser number of partially beamformed signals, generally matching that of the system beamformer, which typically has 128 channels. This means that a 128 conductor cable can be used to couple the partially beamformed signals to the mainframe and its system beamformer, where the 128 channel system beamformer completes the beamforming delay and summation, resulting in one coherent steered and focused signal for the depths covered by the receive beam. U.S. Pat. 5,997,479 (Savord et al.) illustrate how a two dimensional array transducer is beamformed in a typical commercial ultrasound system. The array transducer is divided into groups of contiguous transducer elements, generally ranging from sixteen to one hundred elements. Each group or patch of elements is coupled to a portion of the microbeamformer, termed a subarray by Savord et al. Each subarray beamforms the signals from its patch of transducer elements to a single beamformed signal. One hundred and twenty-eight subarrays thus produce 128 channels of signals which are then combined to a single coherent receive signal by the 128 channels of the system beamformer. WO 2010/055428 A1 describes a beamformer integrated circuit with several microbeamformers together having sixty-four microbeamformer channels, which may be utilized with a 64-element and 128-element array trasnducer. In each microbeamformer the eight signals from the eight transducers are amplified and delayed. These signals further combined to form a single partially beamformed signal, which is further coupled to one of eight inputs of a system beamformer.

Several other implementations of microbeamformer probes have been described in the prior art. U.S. Pat. 6,102,863 (Pflugrath et al.) and U.S. Pat. 6,705,995 (Poland et al.) illustrate the ultimate extension of the microbeamformer, using the probe microbeamformer to do all of the beamforming. Only a single conductor is then needed to coupled the fully beamformed signal from the probe to the system mainframe. Pflugrath et al. connect the probe cable to a connector on the system mainframe which bypasses the system beamformer, applying the fully beamformed signals directly to the system image processor. Poland et al. apply the fully beamformed signal over the probe cable to an A/D converter interface unit, from which the digital signals are applied directly to an electronic display unit.

Another implementation using a microbeamformer is described in U.S. Pat. 7,037,264 (Poland). In this patent the microbeamformer steers a single image plane from the two dimensional array transducer of a 3D imaging probe. The steering of the single plane enables 2D imaging of an image plane which is not orthogonal to the lens of the probe, a capability made possible by the ability of the 3D probe to steer beams in both azimuth and elevation. This makes it possible to steer an image plane through a small acoustic aperture such as the space between the ribs.

In accordance with the principles of the present invention, a diagnostic ultrasound system is operable with a plurality of 2D imaging probes, each of which employs a microbeamformer to partially beamform the signals from the one dimensional (1D) array of transducer elements of the 2D probe down to a small number of partially beamformed signal channels, typically numbering eight to sixteen channels. A system beamformer with eight to sixteen channels, herein termed a minibeamformer, then completes the beamformation to produce fully beamformed coherent echo signals. Each of the 2D imaging probes thus needs a cable with a low number of analog or digital signal paths for the eight to sixteen channels, as compared with the sixty-four or 128 signal paths needed for the typical 2D imaging probe. A mainframe system comprises a probe select signal PS, which is applied to a multiplexer, wherein the multiplexer couples the signal paths of the selected probe and its connector to the inputs of the minibeamformer. Such an architecture enables the same beamformer ICs and printed circuit board to be used in a variety of different probes, such as linear arrays, curved arrays, phased arrays and endocavity
(*e.g.,* endovaginal) transducer (IVT) probes, thereby providing design and manufacturing efficiencies. In accordance with a further aspect of the present invention, the standardized probe transducer ICs can be located in either the handle of the probe or in the connector which connects the probe cable to the system mainframe, the latter enabling an unaltered traditional 2D imaging probe and cable to be used with the inventive microbeamformer architecture.

In the drawings:
FIGURE 1 illustrates a microbeamformer ASIC suitable for use in a 2D imaging probe in accordance with the principles of the present invention.
FIGURE 2 illustrates a 128 element 1D transducer array with probe microbeamformers and a system minibeamformer in accordance with the present invention.
FIGURE 3 illustrates a 160 element 1D transducer array with probe microbeamformers and a system minibeamformer in accordance with the present invention.
FIGURE 4 illustrates an implementation of a legacy 2D imaging probe in accordance with the present invention with the microbeamformer ICs in the probe connector.
FIGURE 5 illustrates a family of different 2D imaging probes, each of which employs the same microbeamformer ASICs, and an ultrasound system mainframe with a minibeamformer constructed in accordance with the principles of the present invention.

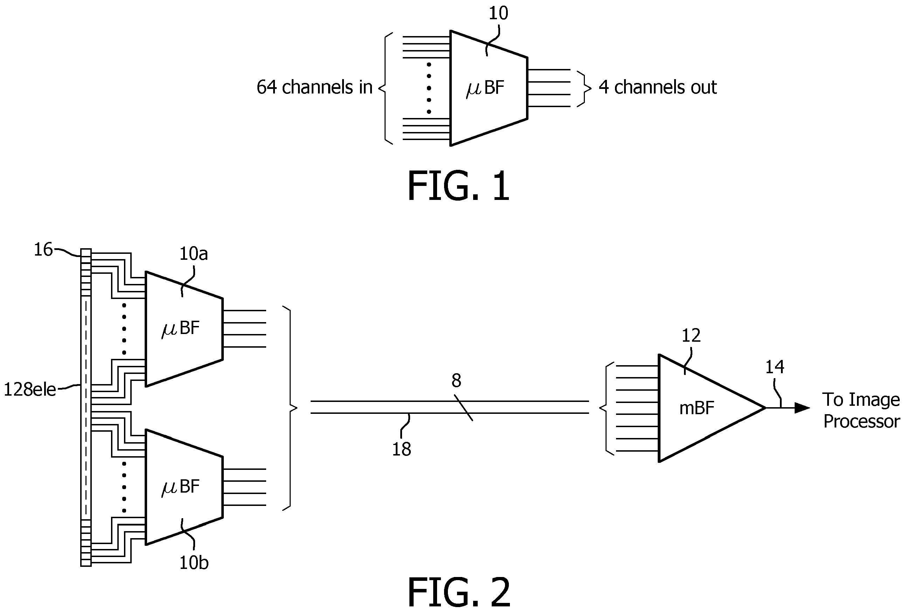

Referring first to FIGURE 1, a probe microbeamformer ASIC 10 suitable for use in a 2D imaging probe in accordance with the principles of the present invention is illustrated in block diagram form. Considering just the receive functionality of the microbeamformer, the ASIC 10 has sixty-four inputs which couple to sixty-four elements of a 1D transducer array. The microbeamformer ASIC controllably delays the signals from the sixty-four transducer elements, then combines them in groups of sixteen input channels. The result is four outputs of partially beamformed signals. In this example each output is the combination of signals from sixteen transducer elements. The microbeamformer ASIC 10 thus reduces sixty-four input channels to four output channels, providing a convenient standard microbeamformer architecture for a variety of 2D imaging probes.

FIGURE 2 illustrates the use of this standard architecture with a system minibeamformer in accordance with the principles of the present invention. In this example two of the standard microbeamformer ASICs, 10a and 10b, are used in the probe with a 128 element 1D transducer array 16.

Half of the 128 elements are coupled to the sixty-four inputs of ASIC 10a, and the other half of the elements are coupled to the sixty-four inputs of ASIC 10b. There are eight partially beamformed output signal paths from the probe, which are coupled to the system mainframe by an 8-conductor probe cable 18. The probe cable may have eight analog signal conductors if the signals are still in analog form, or the digital equivalent thereof if the signals are digitized in the probe. The eight partially beamformed signal paths of the cable 18 are coupled to the eight channels of a minibeamformer 12 in the system mainframe. The minibeamfomer 12 completes the beamformation process, delaying with group delays and combining the eight signals to produce a fully beamformed coherent echo signal at its output 14.

The fully beamformed signals can then be forwarded to subsequent assemblies of the system mainframe for image processing and display. The use of the same micro- beamformer ASIC to reduce the ultrasound signals in the probe and its cable provides a number of advantages. First a standardized microbeamformer ASIC can be used modularly multiple times to perform the probe beamforming. In this example two of the same microbeamformer ASICs are used, providing a standardized approach to beamformation applicable to a variety of different probes. Second, this architecture enables a smaller, lower cost, low power, low channel count main beamformer system, the minibeamformer 12, which in this example has only eight channels. Third, the implementation of a common circuit board design for the standard microbeamformer across several probe models allows rapid deployment of future probe models. Fourth, the probe cable only carries a low number of signal paths, eight in this example, enabling a probe with a thin, lightweight, less costly cable. Another advantageous feature is that the number of pins required in the probe and system connectors can be much less than the maximum number of array elements supported by the system, 128 elements in this example, reducing connector size, cost, and weight.

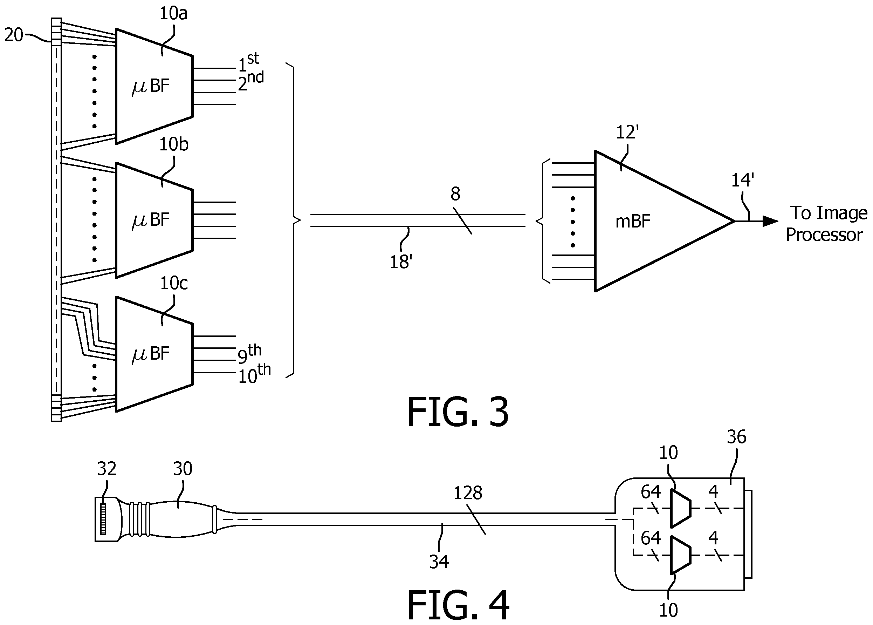

FIGURE 3 illustrates another implementation of a microbeamformer and minibeamformer architecture for a 160 element 1D array 20. The same standard microbeamformer ASIC as before is used in this implementation. In this case three ASICs are used. The ASICs 10a and 10b are each coupled to sixty-four elements of the transducer array 20. The remaining thirty-two elements of the 160-element array 20 are coupled to half the input channels of the third microbeamformer ASIC 10c. Since only half of the third ASIC 10c is effectively used, only two of its four outputs are used. There are a total of ten partially beamformed output signal paths to be coupled from the probe to the system mainframe by way of the probe cable 18'. The 10 partially beamformed output signal paths are reduced to eight by connecting the ones corresponding to the outermost element groups together. Thus the first and second microbeamformer outputs on one end of the sensor array are connected to the 9^{th} and 10^{th} microbeamformer outputs on the other end, respectively. Because only a portion of the acoustic aperture not exceeding 8 element groups is active at any one time, for any scan line position, only 8 signal paths are needed. The microbeamformer ASICs 10a, 10b, and 10c are configured by the system for each scan line to activate contiguous ranges of elements in only 8 of the 10 sub-arrays. As the scan lines progress across a scanned frame, the range of activated elements shifts across the active aperture, and the corresponding microbeamformer outputs are activated accordingly. Thus, an eight conductor cable 18' will suffice for the eight analog signal paths. These eight signal paths are coupled to the eight inputs of the minibeamformer 12' of the mainframe ultrasound system, which completes the beamformation to provide a fully beamformed coherent echo signal at its output 14'. If the 128-element probe of FIGURE 2 were coupled to the minibeamformer 12', its eight analog signal paths would utilize the same 8 channel cable in connecting to the eight input channels of the minibeamformer 12' to complete beamformation for the 128-element array transducer. It is seen that two different probes of the same modular microbeamformer design can be used with the same system mainframe.

FIGURE 4 illustrates another implementation of the present invention for a 2D imaging probe in which the standard microbeamformer ASICs are located in the probe connector which connects the cable to the system mainframe. In this example the probe 30 is a traditional legacy probe with a linear array transducer 32 of 128 transducer elements. Each element of the array is coupled to its own conductor of a 128 conductor cable 34, and the probe cable 34 terminates at a probe connector 36 which connects to a mating connector on an ultrasound system mainframe. The probe and cable, as thus far described, has been commonly available for ultrasound systems for many years. In accordance with the principles of the present invention the standard micro beamformer ASICs 10 previously described are employed in the probe connector 36 to reduce the 128 signal paths of the probe and cable to eight partially beamformed output signal paths. Half of the 128 cable signal paths are coupled to one microbeamformer ASIC, and the other half are coupled to the other ASIC. There are a total of eight partially beamformed signal paths to be coupled to the system beamformer. Thus the legacy probe configuration of FIGURE 4 can be used with either minibeamformer 12 of FIGURE 2 or minibeamformer 12' of FIGURE 3 to complete the beamformation process and produce coherent echo signals suitable for forming an image. Legacy probes using a sixty-four element 1D array transducer will need only one microbeamformer 10 of FIGURE 1, and the cable will need only four signal paths for the received, partially beamformed signals from the microbeamformer.

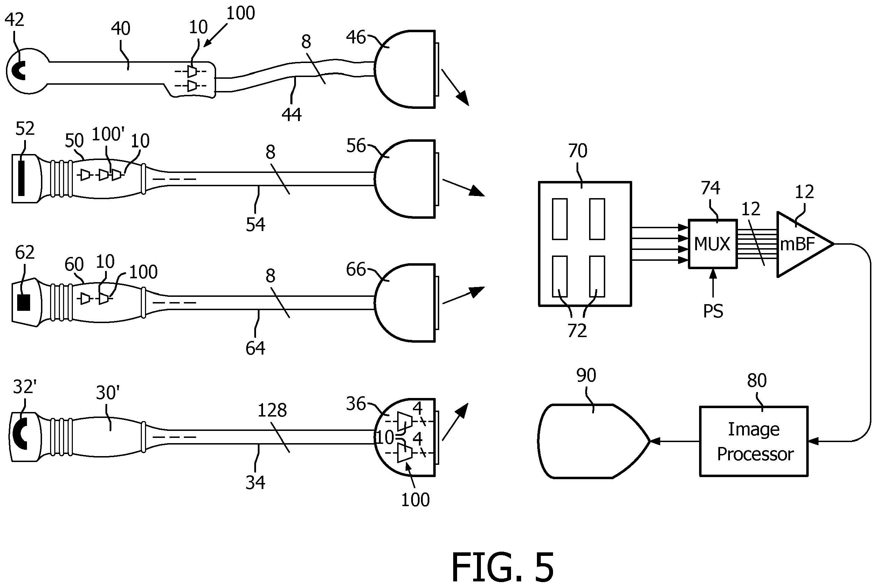

FIGURE 5 illustrates a family of different 2D imaging probes, all of which operate with a reduced channel count system beamformer 12 in accordance with the principles of the present invention. The family of probes includes an endovaginal probe 40 with a 128-element tightly curved array 42, a linear array probe 50 with a 192-element linear array transducer 52, a phased array probe 60 for cardiac imaging with a 128-element phased array transducer 62, and a legacy curved linear probe 30' for obstetrical imaging with a 128-element curved array 32'. The endovaginal probe 40 has an eight signal path cable 44 terminating at a probe connector 46. The linear array probe 50 has an eight signal path cable 54 terminating at a probe connector 56 and utilizes the aforementioned method of connecting microbeamformer outputs to common signal path channels, where in this case the two microbeamformers serving the outer groups of channels in the array have their outputs connected together, sharing a quadruplet of signal paths in the cable. The phased array probe 60 has an eight signal path cable 64 terminating at a probe connector 66. The legacy curved linear probe 30' has a conventional 128 conductor cable 34 terminating at a probe connector 36.

The same standard microbeamformer ASIC 10 is used with each of these probes. For the endovaginal probe 40 two microbeamformer ASICs 10 are mounted on a printed circuit board 100 which is located in the handle of the endovaginal probe. For the 192-element array 52 of the linear array probe 50, three microbeamformer ASICs 10 on p.c.b. 100 are used in the handle of the probe. Each ASIC in the linear array probe is connected to sixty-four of the 192 elements of the array transducer. For the 128 elements of the phased array probe 60, two microbeamformer ASICs on p.c.b. 100 are used in the handle of the probe. The handle of each of these probes comprises the case containing the probe components including the array transducer. For the legacy curved linear array probe, the 128 cable conductors from the 128 elements of the array transducer are coupled to two microbeamformer ASICs on p.c.b. 100 in the probe connector 36. If the interior space of the probe or connector permits, the same p.c.b. can be used for each probe. If the space inside the probe handle or connector is limited, different p.c.b. form factors are used as necessary to fit the probe's space requirements.

Any one or all of the probes of the family may be coupled to identical system probe connectors 72 located on a probe connector block 70 of the system mainframe. The system mainframe may be any ultrasound system that produces an image, including a cart-borne systems and hand-held portable systems. If multiple probes are physically connected, it is necessary to select one of the probes for use. A probe select signal PS is applied to a multiplexer 74 which couples the signal paths of the selected probe and its connector to the inputs of an eight channel minibeamformer 12. The minibeamformer 12 completes beamformation of the eight partially beamformed signals from the microbeamformers of the probe and the coherent echo signals at the minibeamformer output are coupled to an image processor 80 and the formed 2D ultrasound image is displayed on a display 90.

The eight channels of the system minibeamformer are sufficient for any probe of the probe family. When the 192-element linear array probe 50 is selected, which uses three microbeamformer ASICs 10, the eight channels of the minibeamformer are connected to the 12 outputs of the microbeamformer ASICs 10 by connecting 4 of the channels in common. As described above, the system configures the state of the microbeamformer ASICs such that for any scan line, only a maximum of 8 microbeamformer outputs is active, driving the 8 channel inputs of minibeamformer 12. It is seen that a variety of different probes, including legacy probes, can be used with the inventive beamformer architecture, and the system beamformer has a significantly reduced number of channels. Since size, weight, and complexity are all reduced, this architecture is suitable for both cart-borne ultrasound systems and smaller portable or hand-held systems.

Other variations of the implementation of the inventive beamformer architecture will be readily apparent to those skilled in the art. If the system minibeamformer is scaled up to twelve channels, it can accommodate up to three of the 4-output standardized microbeamformers in such a way as to activate and beamform 192 elements simultaneously, rather than as a shifting sub-array of 128 elements. This alternative may provide an advantage for probes with finer pitch elements, to allow larger beamforming apertures, increasing image resolution in the lateral direction. Similarly, if the system minibeamformer comprises 16 channels, 256 transducer elements can be simultaneously beamformed via 4 microbeamformer ASICs. This enables 2D imaging probes with up to 256 simultaneously active transducer elements with the standard ASIC described above. If the family of probes all use transducer sensor arrays with 128 elements or less, an eight channel system beamformer can accommodate the entire family of probes with the ability to beamform all elements simultaneously on any scan line. Alternatively, microbeamformers of greater channel reduction than sixty-four to four can be used. However, it is desirable to select a standard ASIC that can be used for all current probes as well as those envisioned for the future. The microbeamformer ASICs can be flip-chip mounted to the transducer array stack to form a compact assembly that fits inside a small probe case. The transducer array stack can comprise either a piezoelectric ceramic (e.g., PZT) array or a CMUT or PMUT micromachined transducer array made by semiconductor processes.

## Claims

1. An ultrasonic diagnostic system for two dimensional (2D) imaging with a family of 2D imaging probes comprising:
a plurality of 2D imaging probes for different clinical applications, each having an array (20) transducer and one or more of the same microbeamformer (10) coupled to the elements of the array transducer, the one or more microbeamformers each producing from four to sixteen partially beamformed receive signals;
a cable (34,44,54,64) coupled to each of the probes for coupling the partially beamformed receive signals to a mainframe ultrasound system;
a connector (36,46,56,66) at the end of each cable which is adapted to couple to a mainframe ultrasound system; and a mainframe ultrasound system having:
a mating connector adapted to be engaged by a probe cable;
a multiplexer (74), to which a probe select signal (PS) is applied,
a beamformer (12) coupled to receive via the multiplexer (74) signals from the mating connector of the probe selected by the probe select signal (PS), the beamformer having four to sixteen channels for processing partially beamformed receive signals from the selected probe to form fully beamformed receive signals;
an image processor (80) responsive to the fully beamformed receive signals; and
a display (90) coupled to the image processor.

2. The ultrasonic diagnostic system of Claim 1, wherein the microbeamformer further comprises a standard component used in all of the 2D imaging probes.

3. The ultrasonic diagnostic system of Claim 2, wherein the microbeamformer has sixty-four inputs and four outputs at which partially beamformed signals are produced.

4. The ultrasonic diagnostic system of Claim 2, wherein each 2D imaging probe contains a plurality of microbeamformers.

5. The ultrasonic diagnostic system of Claim 1, wherein one of the 2D imaging probes has a probe handle containing the array transducer and the microbeamformer is located in the probe handle.

6. The ultrasonic diagnostic system of Claim 5, wherein the cable contains from four to sixteen receive signal paths coupled to the microbeamformer in the probe handle.

7. The ultrasonic diagnostic system of Claim 1, wherein the microbeamformer is located in the probe connector.

8. The ultrasonic diagnostic system of Claim 1, wherein the mainframe system beamformer has eight to sixteen signal path inputs.

9. The ultrasonic diagnostic system of Claim 8, wherein the mating connector further comprises a probe connector block (70) having a plurality of mating connectors at which the plurality of probes may be simultaneously connected to the mainframe ultrasound system.

10. The ultrasonic diagnostic system of Claim 1, wherein each 2D imaging probe has a 1D array transducer for imaging a two dimensional image plane.

11. The ultrasonic diagnostic system of Claim 10, wherein two of the 1D array transducers comprise two of a curved array, a linear array, a phased array, or a tightly curved array.

12. The ultrasonic diagnostic system of Claim 1, wherein the microbeamformer further comprises a microbeamformer ASIC, and wherein each 2D imaging probe contains at least two microbeamformer ASICs.

13. The ultrasonic diagnostic system of Claim 12, wherein each 2D imaging probe further comprises an array transducer enclosed in a probe case and the microbeamformer ASICs of one of the probes are located in the probe case.

14. The ultrasonic diagnostic system of Claim 12, wherein the microbeamformer ASICs of one of the probes are located in the connector at the end of the cable.

15. The ultrasonic diagnostic system of Claim 1, wherein the array transducer of a probe comprises one of a ceramic piezoelectric array transducer or a micromachined ultrasonic transducer (MUT) array.

## Patentansprüche

1. Diagnostisches Ultraschallsystem zur zweidimensionalen (2D) Bildgebung mit einer Familie von 2D-Bildgebungssonden, umfassend:
eine Vielzahl von 2D-Bildgebungssonden für verschiedene klinische Anwendungen, wobei jede einen Arraywandler (20) und einen oder mehrere von dem gleichen Mikrostrahlformer (10) gekoppelt mit den Elementen des Arraywandlers hat, wobei der eine oder die mehreren Mikrostrahlformer jeweils vier bis sechzehn teilweise strahlgeformte Empfangssignale erzeugen;
ein Kabel (34, 44, 54, 64), das mit jeder der Sonden gekoppelt ist, um die teilweise strahlgeformten Empfangssignale mit einem Hauptultraschallsystem zu koppeln;
einen Konnektor (36, 46, 56, 66) am Ende jedes Kabels, der dafür ausgelegt ist, mit einem Hauptultraschallsystem gekoppelt zu werden; und ein Hauptultraschallsystem mit:
einem passenden Konnektor, der dafür ausgelegt ist, durch ein Sondenkabel in Eingriff gebracht zu werden;
einen Multiplexer (74), an den ein Sondenauswahlsignal (PS) angelegt wird,
einen Strahlformer (12), der gekoppelt ist, um über den Multiplexer (74) Signale von dem passenden Konnektor der durch das Sondenauswahlsignal (PS) ausgewählten Sonde zu empfangen, wobei der Strahlformer vier bis sechzehn Kanäle zum Verarbeiten teilweise strahlgeformter Empfangssignale von der ausgewählten Sonde umfasst, um vollständig strahlgeformte Empfangssignale zu bilden;
einen Bildprozessor (80), der auf die vollständig strahlgeformten Empfangssignale reagiert; und
eine Anzeige (90), die mit dem Bildprozessor gekoppelt ist.

2. Diagnostisches Ultraschallsystem nach Anspruch 1, wobei der Mikrostrahlformer ferner eine Standardkomponente umfasst, die in allen von den 2D-Bildgebungssonden verwendet wird.

3. Diagnostisches Ultraschallsystem nach Anspruch 2, wobei der Mikrostrahlformer vierundsechzig Eingänge und vier Ausgänge hat, an denen teilweise strahlgeformte Signale erzeugt werden.

4. Diagnostisches Ultraschallsystem nach Anspruch 2, wobei jede 2D-Bildgebungssonde eine Vielzahl von Mikrostrahlformern enthält.

5. Diagnostisches Ultraschallsystem nach Anspruch 1, wobei eine der 2D-Bildgebungssonden einen den Arraywandler enthaltenden Sondengriff hat und der Mikrostrahlformer sich in dem Sondengriff befindet.

6. Diagnostisches Ultraschallsystem nach Anspruch 5, wobei das Kabel vier bis sechzehn Empfangssignalpfade enthält, die mit dem Mikrostrahlformer in dem Sondengriff gekoppelt sind.

7. Diagnostisches Ultraschallsystem nach Anspruch 1, wobei sich der Mikrostrahlformer in dem Sondenkonnektor befindet.

8. Diagnostisches Ultraschallsystem nach Anspruch 1, wobei der Hauptsystemstrahlformer acht bis sechzehn Signalpfadeingänge hat.

9. Diagnostisches Ultraschallsystem nach Anspruch 8, wobei der passende Konnektor ferner einen Sondenkonnektorblock (70) mit einer Vielzahl von passenden Konnektoren umfasst, an denen die Vielzahl von Sonden gleichzeitig mit dem Hauptultraschallsystem verbunden werden kann.

10. Diagnostisches Ultraschallsystem nach Anspruch 1, wobei jede 2D-Bildgebungssonde einen 1D-Arraywandler zur Bildgebung einer zweidimensionalen Bildebene hat.

11. Diagnostisches Ultraschallsystem nach Anspruch 10, wobei zwei der 1D-Arraywandler zwei von einem gekrümmten Array, einem linearen Array, einem phasengesteuerten Array oder einem eng gekrümmten Array umfasst.

12. Diagnostisches Ultraschallsystem nach Anspruch 1, wobei der Mikrostrahlformer ferner ein Mikrostrahlformer-ASIC umfasst, und wobei jede 2D-Bildgebungssonde mindestens zwei Mikrostrahlformer-ASICs enthält.

13. Diagnostisches Ultraschallsystem nach Anspruch 12, wobei jede 2D-Bildgebungssonde ferner einen in einem Sondengehäuse eingeschlossenen Arraywandler umfasst und sich die Mikrostrahlformer-ASICs von einer der Sonden in dem Sondengehäuse befinden.

14. Diagnostisches Ultraschallsystem nach Anspruch 12, wobei sich die Mikrostrahlformer-ASICs von einer der Sonden in dem Konnektor am Ende des Kabels befinden.

15. Diagnostisches Ultraschallsystem nach Anspruch 1, wobei der Arraywandler einer Sonde einen von einem keramischen piezoelektrischen Arraywandler oder einem mikrobearbeiteten Ultraschallwandler (MUT)-Array umfasst.

## Revendications

1. Système de diagnostic ultrasonore pour imagerie bidimensionnelle (2D) avec une famille de sondes d'imagerie 2D comprenant :
une pluralité de sondes d'imagerie 2D pour différentes applications cliniques, chacune ayant un transducteur réseau (20) et un ou plusieurs du même formeur de microfaisceau (10) couplés aux éléments du transducteur réseau, chacun de l'un ou plusieurs formeurs de microfaisceau produisant de quatre à seize signaux de réception partiellement formés en faisceau ;
un câble (34, 44, 54, 64) couplé à chacune des sondes pour coupler les signaux de réception partiellement formés en faisceau à un système ultrasonore central ;
un connecteur (36, 46, 56, 66) à l'extrémité de chaque câble qui est apte à être couplé à un système ultrasonore central ; et un système ultrasonore central comprenant :
un connecteur d'accouplement apte à être mis en prise avec un câble de sonde ;
un multiplexeur (74) auquel est appliqué un signal de sélection de sonde (PS) ;
un formeur de faisceau (12) couplé pour recevoir, par l'intermédiaire du multiplexeur (74), des signaux en provenance du connecteur d'accouplement de la sonde sélectionnée par le signal de sélection de sonde (PS), le formeur de faisceau comprenant quatre à seize canaux pour traiter des signaux de réception partiellement formés en faisceau provenant de la sonde sélectionnée pour former des signaux de réception complètement formés en faisceau ;
un processeur d'image (80) répondant aux signaux de réception complètement formés en faisceau ; et
un affichage (90) couplé au processeur d'image.

2. Système de diagnostic ultrasonore selon la revendication 1, dans lequel le formeur de microfaisceau comprend en outre un composant standard utilisé dans toutes les sondes d'imagerie 2D.

3. Système de diagnostic ultrasonore selon la revendication 2, dans lequel le formeur de microfaisceau comprend soixante-quatre entrées et quatre sorties auxquelles des signaux partiellement formés en faisceau sont produits.

4. Système de diagnostic ultrasonore selon la revendication 2, dans lequel chaque sonde d'imagerie 2D contient une pluralité de formeurs de micro faisceau.

5. Système de diagnostic ultrasonore selon la revendication 1, dans lequel l'une des sondes d'imagerie 2D comprend une poignée de sonde contenant le transducteur réseau et le formeur de micro faisceau est situé dans la poignée de sonde.

6. Système de diagnostic ultrasonore selon la revendication 5, dans lequel le câble contient quatre à seize voies de signal de réception couplées au formeur de microfaisceau dans la poignée de sonde.

7. Système de diagnostic ultrasonore selon la revendication 1, dans lequel le formeur de microfaisceau est situé dans le connecteur de sonde.

8. Système de diagnostic ultrasonore selon la revendication 1, dans lequel le formeur de faisceau du système central comprend huit à seize entrées de voie de signal.

9. Système de diagnostic ultrasonore selon la revendication 8, dans lequel le connecteur d'accouplement comprend en outre un bloc de connecteur de sonde (70) comportant une pluralité de connecteurs d'accouplement auxquels la pluralité de sondes peut être simultanément raccordée au système ultrasonore central.

10. Système de diagnostic ultrasonore selon la revendication 1, dans lequel chaque sonde d'imagerie 2D comporte un transducteur réseau 1D pour une imagerie d'un plan d'image bidimensionnel.

11. Système de diagnostic ultrasonore selon la revendication 10, dans lequel deux des transducteurs réseaux 1D comprennent deux d'un réseau incurvé, d'un réseau linéaire, d'un réseau en phase ou d'un réseau étroitement incurvé.

12. Système de diagnostic ultrasonore selon la revendication 1, dans lequel le formeur de microfaisceau comprend en outre un ASIC de formeur de microfaisceau, et dans lequel chaque sonde d'imagerie 2D contient au moins deux ASIC de formeur de microfaisceau.

13. Système de diagnostic ultrasonore selon la revendication 12, dans lequel chaque sonde d'imagerie 2D comprend en outre un transducteur réseau enfermé dans un boîtier de sonde et les ASIC de formeur de microfaisceau de l'une des sondes sont situés dans le boîtier de sonde.

14. Système de diagnostic ultrasonore selon la revendication 12, dans lequel les ASIC de formeur de micro faisceau de l'une des sondes sont situés dans le connecteur à l'extrémité du câble.

15. Système de diagnostic ultrasonore selon la revendication 1, dans lequel le transducteur réseau d'une sonde comprend l'un d'un transducteur réseau piézo-électrique céramique ou d'un réseau de transducteurs ultrasonores micro-usinés (MUT).
